# EUROPÄISCHE PATENTSCHRIFT

(11) **EP 3 251 641 B1**
(45) Veröffentlichungstag und Bekanntmachung des Hinweises auf die Patenterteilung: **06.01.2021**
(21) Anmeldenummer: 16172601.3
(22) Anmeldetag: 02.06.2016
(51) Int. Cl.: A61F 13/08, A61F 5/01, D04B 1/26, D04B 1/18, D04B 21/18

(54) **KOMPRESSIONSARTIKEL**
COMPRESSION ARTICLE
ARTICLE DE COMPRESSION

(43) Veröffentlichungstag der Anmeldung: 06.12.2017
(73) Patentinhaber: BSN-Jobst GmbH, 46446 Emmerich (DE)
(72) Erfinder: BAUER, Joachim, 22761 Hamburg (DE); FRANKENBERG, Bernhard, 37351 Silberhausen (DE); PLATZ, Sascha, 47533 Kleve (DE)
(74) Vertreter: Nederlandsch Octrooibureau

(56) Entgegenhaltungen:
- EP-A1- 1 213 002
- US-A- 4 961 418
- US-A1- 2005 113 729
- US-A1- 2008 125 688

## Beschreibung

Kompressionsartikel dienen dem Bandagieren von Gliedmaßen und/oder Gelenken. Sie werden außerdem zur Behandlung von Ödemen eingesetzt.

Zur Ödembehandlung sind insbesondere Bein- oder Armstrümpfe, aber auch Handschuhe mit und ohne Finger im Einsatz. Diese Artikel werden in der Regel nur tagsüber getragen. Sie sind mittlerweile durchaus modisch gestaltet und fallen kaum noch als medizinische Produkte auf. Die gebräuchlichen Kompressionsartikel sind teilweise der Patientenanatomie sehr gut angepasst bzw. passen sich dieser sehr gut an und können einen patientenindividuellen Kompressionsdruck und Kompressionsdruckverlauf aufweisen. Dadurch sind diese Produkte sicher in der Anwendung und gewährleisten die therapeutische Wirksamkeit.

Schwerere Ödeme werden insbesondere nachts dadurch behandelt, dass die betroffenen Extremitäten mit Bandagen zur Kompressionstherapie umwickelt werden. Das Bandagieren wird meist von den Patienten selbst durchgeführt, was ein gewisses Geschick und Erfahrung erfordert. Die Bandagen werden meist mehrlagig aufgebracht, wobei die unterschiedlichen Lagen unterschiedliche Funktionen übernehmen. Eine erste Lage ist meist eine Hautschutz- und Polster/Druckverteilungslage, eine weitere die eigentliche Kompressionslage. Die Kompressionstherapie ist nur dann wirksam, wenn die Bandagen der Kompressionslage straff genug angelegt werden, sodass die gestaute Flüssigkeit in die Lymphgefäße und in die Venen abfließen kann. Andererseits muss aber darauf geachtet werden, dass es beim Bandagieren zu keinen Einschnürungen an den Gliedmaßen kommt und ein erholsamer Schlaf weiterhin möglich ist. Vielfach werden Bandagen aus therapeutischer Sicht mit zu geringer Kompression oder ungünstigen Kompressionsverläufen entlang des Gliedmaßes und damit falsch angelegt.

Bei Verletzungen sind herkömmliche Kompressionsartikel ebenfalls nur bedingt einsetzbar, da offene Wunden sehr empfindlich für Druckspitzen sind. Ebenso müssen knöcherne und andere Erhebungen an den Gliedmaßen vor Druckspitzen geschützt werden.

Aus der US 4 961 418 A ist ein therapeutischer Artikel zur Behandlung von Verletzungen bekannt, der drei Lagen aufweist, wobei die mittlere Lage eine Schaumstofflage ist. Die innerste Lage soll die Zirkulation in der Haut erhöhen. Über einen Klettverschluss kann der Artikel mit dem gewünschten Druck an einem Körperteil festgelegt werden.

Die US 2008/0125688 A1 und die US 2005/0113729 A1 beschreiben Kompressionsartikel zur Drainage von überschüssigen Körperflüssigkeiten aus einem Körperteil. Die Artikel weisen ebenfalls zwei äußere Lagen und eine dazwischen angeordnete, polsternde Schaumstofflage auf. Der Kompressionsdruck kann durch Verschlusseinrichtungen verändert werden.

In der EP 1 213 002 A1 ist ein Kompressionsartikel mit einer Zwischenlage mit aufblasbaren Zellen bekannt. Durch Variation der Luftmenge in den Zellen kann hier der Kompressionsdruck verändert werden.

Der vorliegenden Erfindung liegt die Aufgabe zugrunde, einen Kompressionsartikel bereitzustellen, der eine verbesserte Verteilung des Kompressionsdruckverlaufs sowie eine bessere Polsterung ermöglicht als bekannte Kompressionsartikel und der auch derart gestaltet werden kann, dass er als Ersatz für das Bandagieren zur Kompressionstherapie einsetzbar ist.

Die Aufgabe wird gelöst durch einen Kompressionsartikel mit den Merkmalen des Patentanspruchs 1.

Der Kompressionsartikel kann ein im Wesentlichen schlauchförmiges Gebilde sein, das über das zu stützende Körperteil ziehbar ist. Der Kompressionsartikel kann dazu beispielsweise als Arm- oder Beinstrumpf, als Handschuh oder als Bandage ausgebildet sein und einfach über das entsprechende Körperteil gezogen werden.

Alternativ oder zusätzlich kann der Kompressionsartikel über eine Verschlusseinrichtung verfügen, mit der er an dem zu stützenden oder komprimierenden Körperteil festlegbar ist. Als Verschlusseinrichtungen kommen beispielsweise Reißverschlüsse, Klettverschlüsse, Riemen, Bänder oder aufrollbare Schnüre oder Drähte oder Kombinationen daraus in Frage, die in Umfangsrichtung z.B. distal am Kompressionsartikel angeordnet sind. Die Verschlusseinrichtungen können dabei auch derart gestaltet sein, dass mit ihrer Hilfe der Kompressionsartikel unterschiedlich straff angelegt werden kann. Dies ist insbesondere dann von Vorteil, wenn der Kompressionsartikel zur Kompressionstherapie nach den Grundsätzen der komplexen physikalischen Entstauungstherapie eingesetzt werden soll. Mit zunehmendem Ausleiten der Gewebsflüssigkeit verringert sich der Umfang des Körperteils. Mit Hilfe der Verschlusseinrichtung lässt sich dies ausgleichen, sodass der gewünschte Kompressionsdruck durch den Kompressionsartikel aufrechterhalten werden kann. Ferner kann durch Öffnen der Verschlusseinrichtung das Anziehen des Artikels erleichtert werden, beim Wiederschließen wird der vorgesehene Kompressionsdruck wieder hergestellt.

Durch die beiden Deckschichten und die verbindende Zwischenlage lassen sich sehr viel stärkere Variationen des Kompressionsdrucks und der Kompressionscharakteristik - der Veränderung des Kompressionsdrucks durch Dehnung des Materials (Elastizitätsmodul) - realisieren als bei einlagigen Kompressionsartikeln. Die Elastizität bzw. der Elastizitätsverlauf der beiden Deckschichten kann unterschiedlich sein. Die verbindende Zwischenlage kann eine geringere oder eine höhere Elastizität und/oder einen abweichenden Elastizitätsverlauf als die Deckschichten aufweisen. Die für die Kompressionstherapie von Ödemen erforderlichen Druckverteilungen und gewünschten Kompressionscharakteristiken können damit problemlos nachgebildet werden.

Sind Verschlusseinrichtungen vorgesehen, so kann mit diesen der Kompressionsdruck eingestellt werden, wobei Hilfsmittel, insbesondere Markierungen, vorgesehen sind, die die Druckeinstellung erleichtern. Dies ist insbesondere dann von Vorteil, wenn Ödeme behandelt werden sollen, die eine schrittweise Erhöhung des Kompressionsdruckes erfordern. Neben Markierungen können auch Beschriftungen oder mit einer Anzeigeeinrichtung versehene Druckmesseinrichtungen vorgesehen sein.

Im entspannten Zustand des Kompressionsartikels können die Deckschichten vorzugsweise zwischen 0,5 mm und 5 mm dick sein und die Zwischenlage zwischen 1 mm und 20 mm. Das Verhältnis der Dicke der Zwischenlage zu der Dicke einer Deckschicht kann bis zu 40 betragen. Besonders bewährt haben sich Deckschichten mit einer Dicke von 0,5 bis 3 mm und eine Zwischenlage mit einer Dicke von 2 bis 10 mm, ganz besonders bewährt haben sich Deckschichten mit einer Dicke von 0,5 bis 2 mm und eine Zwischenlage mit einer Dicke von 3 bis 7 mm. Wenn die Dicke der Zwischenschicht über den Artikel variiert, so wird die größte Dicke innerhalb der Struktur zur Dickenbestimmung herangezogen.

Die Dicke des Artikels unter Druckbelastung kann sich im Laufe des Gebrauchs durch Materialermüdung verändern. Nach 1000 Prüfzyklen kann die plastische, d.h. bleibende Verformung des Artikels vorzugsweise im Bereich von maximal 60%, bevorzugt von maximal 50% und besonders bevorzugt von maximal 40% und die elastische Verformung im Bereich von wenigstens 5% bis 100%, bevorzugt von mindestens 10% bis 100% und besonders bevorzugt von mindestens 15% bis 100% liegen. Dabei wird die Anfangs- und Enddicke einer Probe nach DIN EIN ISO 5084 bestimmt. Dazwischen wird die Probe je Prüfzyklus für eine Minute mit einer Flächenlast von 3,82 N/cm2 belastet. Die Kraftreaktion des Artikels auf die Druckbelastung verläuft vorzugsweise (im mathematischen Sinne) stetig, die Kraft steigt kontinuierlich mit dem Eindrücken des Materials an. Kleinere Bereiche von max. 25% Eindrückungsweg, bevorzugt jedoch 15%, gemessen an der Ausgangsdicke, über die Kraft nicht ansteigt, können akzeptiert werden.

Die Steifigkeit des Kompressionsartikels in Umfangsrichtung kann vorzugsweise zwischen 2 bis 30 mm Hg/cm betragen, wobei der Druckanstieg bei einer Vergrößerung des Umfangs um 1 cm gemessen wird. Bevorzugt beträgt die Steifigkeit 3 bis 20 mmHg/cm und besonders bevorzugt 4 bis 15 mmHg/cm und ganz besonders bevorzugt 4 bis 9 mmHg/cm. Um die Steifigkeit zu ermitteln, werden in Anlehnung an die RAL-GZ 387 jeweils 6cm breite und 24cm lange Streifen des Strickmaterials in Querdehnungsrichtung mit einer Einspannlänge von 20cm in der Zwick-Zugprüfmaschine eingespannt und mit einer Geschwindigkeit von 200mm/min jeweils 6 Mal um 20% gedehnt. Zur Berechnung der für die Steifigkeit maßgeblichen Kompressionswerten wird der Kraftwert im 6ten Zug-Zyklus bei 15% Dehnung und bei 20% Dehnung mit den jeweiligen Körpermaßen 23cm bzw. 24cm verwendet. Die Steifigkeit ist die Differenz der beiden Werte.

Mindestens eine der Deckschichten kann vorzugsweise aus einem textilen Material hergestellt sein. Weist diese zur Haut des Trägers, so ergibt sich ein vergleichsweise hoher Tragekomfort des Kompressionsartikels.

Die Zwischenlage kann fest mit mindestens einer der Deckschichten verbunden oder auch in mindestens eine der Deckschichten integriert sein. Hierdurch ergibt sich ein fester Verbund aus den verschiedenen Lagen.

Weitere Vorteile ergeben sich, wenn die Zwischenlage mindestens teilweise aus Monofilamenten hergestellt ist. Monofilamentfäden sind auf relativ günstige Weise in der gewünschten Qualität, insbesondere hinsichtlich ihrer Biegesteifigkeit und Verarbeitbarkeit, herstellbar.

Um den erforderlichen Kompressionsdruck zu erzeugen bzw. auf das Körperteil übertragen zu können, können die Monofilamente vorzugsweise in einem Winkel zwischen 30° und 90°, bevorzugt zwischen 45° und 90° zu den Deckschichten angeordnet sein. In Versuchen haben sich Winkel zwischen 65° und 85° als besonders vorteilhaft erwiesen.

Die Monofilamente können alle aus dem gleichen Material oder aus verschiedenen Materialien, vorzugsweise aus Polyester, Polyamid oder Polyolefin oder anderen extrudierbaren, thermoplastischen Materialien hergestellt sein.

Der Titer der einzelnen Monofilamente kann zwischen 10 dtex und 2240 dtex betragen. Besonders vorteilhaft sind Titer zwischen 22 und 1000 dtex, besonders bevorzugt sind Titer zwischen 40 und 500 dtex. Die Monofilamente können dabei einzeln oder gefacht oder gezwirnt verarbeitet werden, sodass die resultierende Materialstärke anwendungsbezogen, auch in einem Artikel, variiert werden kann. Der Querschnitt der Monofilamente kann rund sein oder andere Querschnittsformen aufweisen, die in geeigneter Weise das Flächenträgheitsmoment der Monofile beeinflussen, z.b. Trilobal. Ferner können die individuellen Monofilamente aus mehreren Materialien, z.b. als Bicomponentenmonofilament mit einer side-by-side oder einer sheath-core Anordnung hergestellt sein. Die Materialien können hierbei unterschiedliche Eigenschaften aufweisen, z.B. unterschiedliche Schmelzpunkte oder unterschiedliche molekulare Orientierungen oder Orientierungs- bzw. Kristallisationsgrade aufweisen. Durch späteres Erwärmen des Artikels kann damit z.B. an den Berührpunkten der Monofilamente untereinander eine Verklebung erreicht werden, die eine Erhöhung der Druckstabilität des Materials bewirkt.

Der Kompressionsartikel ist aus einem Gestrick oder Gewirk hergestellt, das wenigstens bereichsweise als Abstandsgestrick oder -gewirk ausgebildet ist, wobei die obere und untere Gestricklage die Deckschichten bilden und die Polfäden die Zwischenlage.

Das Abstandsgestrick oder -gewirk kann komplett auf einer Strick- oder Wirkmaschine produziert werden. Das Abstandsgestrick oder -gewirk hat außerdem einen polsternden und druckverteilenden Effekt. Somit lassen sich auch Gliedmaßen mit Haut- oder Gewebeverletzungen noch mit einem solchen Kompressionsartikel versorgen. Der Kompressionsartikel kann dabei vollständig als Abstandsgestrick ausgebildet sein oder nur bereichsweise. Bei Armstrümpfen hat sich im Hand- oder Fußbereich ein Anteil von ca. 20% an Abstandsgestricken oder mehr und im Arm- oder Beinbereich von ca. 40% bewährt. Bevorzugt wird die Gestaltung mit ca. 50% oder mehr im Hand- und Fußbereich und mit 80 bis 100% im Armoder Beinbereich. Andere Ausführungen mit anderen Anteilen des Abstandsgestricks am Gesamtprodukt für andere Körperteile, z. B. den Torso oder den Genitalbereich, sind ebenfalls möglich. Der Kompressionsartikel bedeckt dabei sinnvollerweise bis zu 100% des zu behandelnden Gliedmaßes, kann aber auch lediglich einen Teil des Gliedmaßes bedecken, z.B. um Kombinationen mit anderen Kompressionsprodukten zu ermöglichen. Bevorzugt deckt er aber mindestens 30% der Länge des Gliedmaßes (Arm, Bein) ab. Besonders bevorzugt kann es aber auch bis zu 50% des Körperteils in dessen Längsrichtung abdecken.

Die Polfäden können dabei zumindest teilweise elastische Fäden sein. Soll dagegen die Druckstabilität des Kompressionsartikels zumindest in einigen Bereichen erhöht werden, können auch nicht elastische Polfäden verwendet werden. Die Polfäden können aus Monofilamenten gebildet sein, wobei mehrere Monofilamente parallel in einer Bindung verstrickt werden können.

Weitere Vorteile lassen sich erzielen, wenn der Abstand der beiden Deckschichten über die Oberfläche des Kompressionsartikels variiert. Der Kompressionsartikel liegt dann nicht mehr vollflächig auf dem Körperteil auf, sondern nur in den Bereichen, in denen der Abstand der Deckschichten größer ist, das heißt das Abstandsgestrick eine kissenartige Struktur hat. Dadurch lassen sich Massageeffekte der Haut und des Gewebes erzielen. In einigen Bereichen des Kompressionsartikels kann auch der Auflagedruck verringert werden, wodurch empfindliche Körperstellen geschont werden können und sich der Tragekomfort des Kompressionsartikels erhöht.

Vorzugsweise kann das Abstandsgestrick dabei durch den variierenden Abstand der beiden Deckschichten eine regelmäßige, z.B. waffelartige oder rautenartige Struktur aufweisen. Die Struktur kann jedoch auch unregelmäßig sein.

Bei einer bevorzugten Ausgestaltung des Kompressionsartikels sind zwischen die Deckschichten Schussfäden und/oder Kettfäden eingelegt oder an mindestens eine Deckschicht angebunden. Die Schussfäden können dabei zumindest teilweise elastische Fäden sein. Mit Hilfe der Schussfäden lässt sich der Kompressionsdruck des Artikels weiter regulieren. Mit derselben Bindung und demselben Fadenmaterial für die Deckschichten lassen sich somit Kompressionsartikel mit unterschiedlichen Druckeigenschaften herstellen.

Ist der Kompressionsartikel ein auf einer Flachstrickmaschine hergestelltes Gestrick, so ergeben sich hinsichtlich der Formgebung, Musterung und Bindungsart die meisten Freiheitsgrade bei der Herstellung des Artikels. Er kann auch maßgenau dem Körper eines Patienten angepasst werden.

Vorzugsweise kann der Kompressionsartikel dabei eine dreidimensionale Struktur aufweisen und dadurch dem zu stützenden Körperteil in der Form angepasst werden. Durch ein vorab festgelegtes Verhältnis zwischen den Körperteilmaßen und den Maßen des Kompressionsartikels kann in Abhängigkeit von der Elastizitätscharakteristik des Materials ein vorab festgelegter Kompressionsdruck oder Kompressionsdruckbereich und Kompressionsdruckverlauf erzielt werden. Die dabei notwendige Dehnung des Materials in Umfangsrichtung sollte bevorzugt bei 5% oder mehr liegen um die notwendige Flexibilität bei der Anwendung sicherzustellen. Bevorzugt überschreitet die zur Erreichung der Kompression notwendige Dehnung 100% nicht, um das Anziehverhalten nicht negativ zu beeinflussen. Besonders vorteilhaft ist eine Dehnung, die zur Erreichung des festgelegten Kompressionsdruckbereiches notwendig ist, in Umfangsrichtung von 2% bis 50% und ganz besonders bevorzugt von 5% bis 25%.

Die Elastizität des Materials/des Artikels in Längsrichtung des Gliedmaßes liegt, gemessen nach RAL GZ 387 (Ausgabe 1 oder 2 entsprechend des Styles) zwischen 5% und 200%. Bevorzugt kann die Längselastizität 20% bis 100% und besonders bevorzugt 30% bis 75% betragen. Die Elastizität des Materials kann partiell eingeschränkt werden (z.b. durch Applikationen oder Laminierung mit weiteren Materialien oder Anpassung des Gestricks).

Das Abstandsgestrick kann sich bevorzugt über den gesamten Umfang des Kompressionsartikels erstrecken. Außerdem kann der Kompressionsartikel quer- und längselastisch sein, um den gewünschten Tragekomfort und die gewünschte therapeutische Wirkung zu erzielen. Vorzugsweise wird der Artikel dabei derart gestrickt, dass die Maschenreihenrichtung der Deckschichten der Umfangsrichtung des Kompressionsartikels entspricht.

Im Folgenden werden bevorzugte Ausführungsbeispiele erfindungsgemäßer Kompressionsartikel mit Bezug auf die Zeichnung näher beschrieben.

### Es zeigen:

- Fig. 1: eine schematische Ansicht eines Beinstrumpfes;
- Fig. 2: eine Schnittansicht durch das Gestrick des Beinstrumpfes aus Fig. 1;
- Fig. 3: eine schematische Ansicht einer Kniebandage mit Reißverschluss.

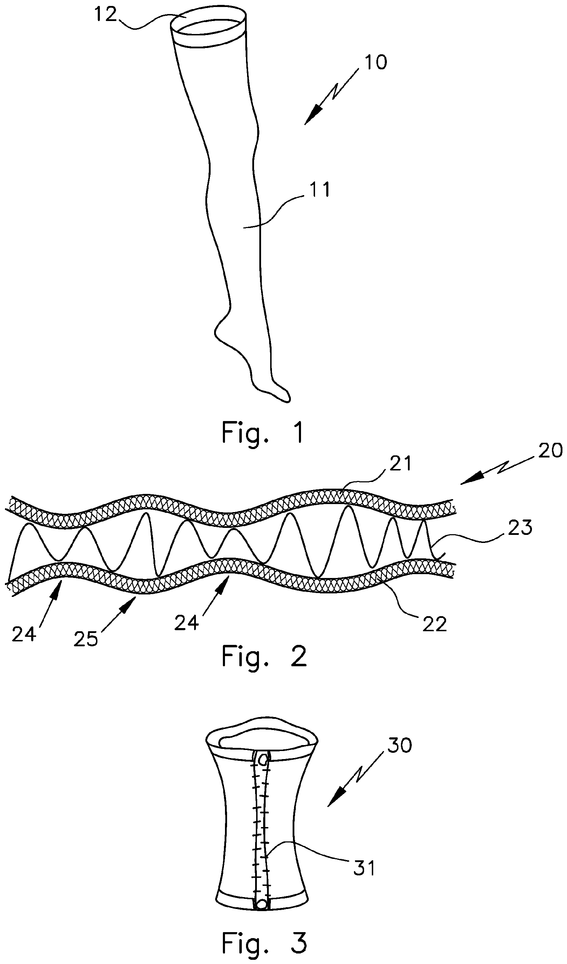

Der in Fig. 1 dargestellte Beinstrumpf 10 dient der Behandlung eines Unterschenkelödems und kann vorzugsweise für das Bein des Patienten maßgefertigt sein. Er besteht aus einem dreidimensionalen Schlauchgestrick 11, an dessen oberes Ende ein Halteband 12 angenäht ist. Aufgrund der Elastizität des Schlauchgestricks 11 und des Haltebands 12 kann der Beinstrumpf 10 einfach über das Bein übergezogen werden.

Das Schlauchgestrick 11 ist als Abstandsgestrick 20 ausgebildet, das in Fig. 2 in einer prinzipiellen Querschnittsdarstellung abgebildet ist. Es weist eine äußere Deckschicht 21 und eine innere Deckschicht 22 auf. Beide Deckschichten 21, 22 sind unter Verwendung elastischer Fäden hergestellt. Die innere Deckschicht 22, die mit der Haut des Trägers des Kompressionsartikels 10 in Kontakt kommt, kann außerdem Fäden aus hautfreundlichem Material wie Baumwolle enthalten. Für die äußere Deckschicht 21 lassen sich dagegen auch Fäden mit hoher Abriebfestigkeit einsetzen. Die beiden Deckschichten 21, 22 sind durch einen Polfaden 23 miteinander verbunden, der vorzugsweise ebenfalls elastisch sein kann. Die Verbindung durch den Polfaden ist dabei derart gewählt, dass Bereiche 24 geringeren Abstands zwischen den Deckschichten 21, 22 entstehen, in denen die innere Deckschicht nicht oder nur mit geringem Druck auf der Haut des Trägers aufliegt. Die zwischen den Bereichen 24 liegenden Bereiche 25 größeren Abstands zwischen den beiden Deckschichten üben dagegen einen Massageeffekt auf die Haut und das Gewebe des Trägers aus und unterstützen somit die therapeutische Wirkung des Kompressionsartikels 10.

In Fig. 3 ist eine alternative Ausgestaltung eines Kompressionsartikels 30 in Form einer Kniebandage gezeigt. Auch die Kniebandage 30 ist zumindest bereichsweise aus einem Abstandsgestrick 20 gemäß Fig. 2 gefertigt. Im Gegensatz zum Kompressionsartikel 10 ist sie jedoch nicht schlauchförmig ausgebildet, sondern besitzt auf ihrer Rückseite einen Reißverschluss 31, mit dessen Hilfe die Kniebandage 30 am Bein des Patienten angelegt werden kann.

Die dargestellten Kompressionsartikel 10, 30 sind lediglich beispielhaft. Sie können auch andere Formen und/oder Verschlusstechniken aufweisen. Auch das Abstandsgestrick 20 ist nur ein mögliches Ausführungsbeispiel. Es könnte auch ein Gewirk eingesetzt werden. Anstelle des Polfadens kann auch eine Zwischenschicht zum Beispiel aus einem Kunststoffschaum zur Verbindung der beiden Deckschichten 21, 22 vorgesehen werden.

## Patentansprüche

1. Kompressionsartikel, der mindestens bereichsweise zwei aus zumindest teilweise elastischem Material hergestellte Deckschichten (21, 22) aufweist, die durch eine Zwischenlage (23) miteinander verbunden sind, wobei mittels der Deckschichten (21, 22) ein Kompressionsdruck erzeugbar ist und die Zwischenlage (23) mindestens die doppelte Dicke wie eine der Deckschichten oder die dünnere der Deckschichten (21, 22) aufweist und der mindestens bereichsweise aus einem komprimierbaren Material gefertigt ist, wobei der Kompressionsartikel über das zu stützende oder zu komprimierende Körperteil ziehbar ist und/oder über eine Verschlusseinrichtung (31) verfügt, mit der er an dem zu stützenden oder komprimierenden Körperteil festlegbar ist, sodass er im angelegten Zustand ein im Wesentlichen schlauchförmiges Gebilde ist, **dadurch gekennzeichnet, dass** er aus einem Gestrick (11) oder Gewirk hergestellt ist, das wenigstens bereichsweise als Abstandsgestrick (20) oder -gewirk ausgebildet ist, wobei die obere und untere Gestricklage die Deckschichten (21, 22) bilden und die Polfäden die Zwischenlage (23).

2. Kompressionsartikel nach Anspruch 1, **dadurch gekennzeichnet, dass** mit der Verschlusseinrichtung (31) der Kompressionsdruck einstellbar ist, wobei Hilfsmittel, insbesondere Markierungen, vorgesehen sind, die die Druckeinstellung erleichtern.

3. Kompressionsartikel nach Anspruch 1 oder 2, **dadurch gekennzeichnet, dass** mindestens eine der Deckschichten (21, 22) aus einem textilen Material hergestellt ist.

4. Kompressionsartikel nach einem der vorhergehenden Ansprüche, **dadurch gekennzeichnet, dass** die Zwischenlage (23) fest mit mindestens einer der Deckschichten (21, 22) verbunden ist oder in mindestens eine der Deckschichten (21, 22) integriert ist.

5. Kompressionsartikel nach einem der vorhergehenden Ansprüche, **dadurch gekennzeichnet, dass** die Zwischenlage (23) mindestens teilweise aus Monofilamenten hergestellt ist.

6. Kompressionsartikel nach Anspruch 5, **dadurch gekennzeichnet, dass** die Monofilamente im entspannten Zustand des Kompressionsartikels in einem Winkel zwischen 30° und 90° zu den Deckschichten (21, 22) angeordnet sind.

7. Kompressionsartikel nach Anspruch 5 oder 6, **dadurch gekennzeichnet, dass** alle Monofilamente aus dem gleichen Material oder aus verschiedenen Materialien, vorzugsweise aus Polyester, Polyamid oder Polyolefin, hergestellt sind.

8. Kompressionsartikel nach einem der vorhergehenden Ansprüche, **dadurch gekennzeichnet, dass** die Polfäden (23) zumindest teilweise elastische Fäden sind.

9. Kompressionsartikel nach einem der vorhergehenden Ansprüche, **dadurch gekennzeichnet, dass** der Abstand der beiden Deckschichten (21, 22) über die Oberfläche des Kompressionsartikels (10, 30) variiert.

10. Kompressionsartikel nach Anspruch 9, **dadurch gekennzeichnet, dass** das Abstandsgestrick (20) durch den variierenden Abstand der beiden Deckschichten (21, 22) eine waffelartige oder rautenartige Struktur aufweist.

11. Kompressionsartikel nach einem der vorhergehenden Ansprüche, **dadurch gekennzeichnet, dass** zwischen die Deckschichten (21, 22) Schuss- und/oder Kettfäden eingelegt oder an mindestens eine Deckschicht angebunden sind.

12. Kompressionsartikel nach Anspruch 11, **dadurch gekennzeichnet, dass** die Schussfäden (21, 22) zumindest teilweise elastische Fäden sind.

13. Kompressionsartikel nach einem der vorhergehenden Ansprüche, **dadurch gekennzeichnet, dass** sich das Abstandsgestrick (20) über den gesamten Umfang des Kompressionsartikels (10, 30) erstreckt.

14. Kompressionsartikel nach einem der vorhergehenden Ansprüche, **dadurch gekennzeichnet, dass** die Maschenreihenrichtung der Deckschichten (21, 22) der Umfangsrichtung des Kompressionsartikels entspricht.

15. Kompressionsartikel nach einem der vorhergehenden Ansprüche, **dadurch gekennzeichnet, dass** durch ein vorab festgelegtes Verhältnis zwischen den Körperteilmaßen und den Maßen des Kompressionsartikels ein vorab festgelegter Kompressionsdruck oder Kompressionsdruckbereich und Kompressionsdruckverlauf erzielbar ist.

## Claims

1. Compression article which has, at least in regions, two cover layers (21, 22) which are made of at least partially elastic material and are connected to one another by an intermediate layer (23), whereby a compression pressure can be generated by means of the cover layers (21, 22) and the intermediate layer (23) is at least twice as thick as one of the cover layers or the thinner of the cover layers (21, 22), and whereby the compression article is made at least in part of a compressible material, whereby the compression article can be pulled over the body part to be supported or compressed and/or the compression article has a closure device (31) by means of which it can be fixed to the body part to be supported or compressed, so that in the applied state it is a substantially tubular structure, **characterized in that** it is made from a weft-knitted fabric (11) or warp-knitted fabric that is formed at least in part as a weft-knitted spacer fabric (20) or warp-knitted spacer fabric, the upper and lower knitted layers forming the cover layers (21, 22) and the pile threads forming the intermediate layer (23).

2. Compression article according to claim 1, **characterized in that** the compression pressure can be adjusted by means of the closure device (31), with aids, in particular markings, being provided which make it easier to adjust the pressure.

3. Compression article according to either claim 1 or claim 2, **characterized in that** at least one of the cover layers (21, 22) is made of a textile material.

4. Compression article according to any of the preceding claims, **characterized in that** the intermediate layer (23) is firmly connected to at least one of the cover layers (21, 22) or is integrated into at least one of the cover layers (21, 22).

5. Compression article according to any of the preceding claims, **characterized in that** the intermediate layer (23) is made at least in part of monofilaments.

6. Compression article according to claim 5, **characterized in that**, in the relaxed state of the compression article, the monofilaments are arranged at an angle between 30° and 90° with respect to the cover layers (21, 22).

7. Compression article according to either claim 5 or claim 6, **characterized in that** all the monofilaments are made of the same material or of different materials, preferably of polyester, polyamide or polyolefin.

8. Compression article according to any of the preceding claims, **characterized in that** the pile threads (23) are, at least in part, elastic threads.

9. Compression article according to any of the preceding claims, **characterized in that** the distance between the two cover layers (21, 22) varies over the surface of the compression article (10, 30).

10. Compression article according to claim 9, **characterized in that** the knitted spacer fabric (20) has a wafer-like or diamond-like structure due to the varying distance between the two cover layers (21, 22).

11. Compression article according to any of the preceding claims, **characterized in that** weft and/or warp threads are inserted between the cover layers (21, 22) or are connected to at least one cover layer.

12. Compression article according to claim 11, **characterized in that** the weft threads (21, 22) are, at least in part, elastic threads.

13. Compression article according to any of the preceding claims, **characterized in that** the - knitted spacer fabric (20) extends over the entire periphery of the compression article (10, 30).

14. Compression article according to any of the preceding claims, **characterized in that** the stitch course direction of the cover layers (21, 22) corresponds to the peripheral direction of the compression article.

15. Compression article according to any of the preceding claims, **characterized in that** a predetermined compression pressure or compression pressure range and compression pressure curve can be achieved by a predetermined ratio between the body part dimensions and the dimensions of the compression article.

## Revendications

1. Article de compression présentant, au moins par endroits, deux couches de recouvrement (21, 22) fabriquées au moins partiellement à partir d'une matière élastique, lesquelles couches de recouvrement sont reliées l'une à l'autre par un élément intermédiaire (23), une pression de compression pouvant être générée au moyen des couches de recouvrement (21, 22) et l'élément intermédiaire (23) étant au moins deux fois plus épais que l'une des couches de recouvrement ou que la plus fine des couches de recouvrement (21, 22), et l'article de compression étant constitué au moins par endroits d'une matière compressible, l'article de compression pouvant recouvrir la partie du corps à supporter ou à comprimer et/ou disposant d'un dispositif de fermeture (31) au moyen duquel il peut être fixé à la partie du corps à supporter ou à comprimer, de manière à se présenter, à l'état placé, sous la forme d'une structure essentiellement tubulaire, **caractérisé en ce qu'**il est fabriqué à partir d'un tricot à mailles cueillies (11) ou d'un tricot à mailles jetées, qui est formé au moins par endroits comme tricot d'espacement (20), les éléments supérieur et inférieur de tricotage formant les couches de recouvrement (21, 22) et les fils de poils formant l'élément intermédiaire (23).

2. Article de compression selon la revendication 1, **caractérisé en ce que** la pression de compression peut être réglée au moyen du dispositif de fermeture (31), des moyens d'aide, en particulier des marquages, étant prévus pour faciliter le réglage de la pression.

3. Article de compression selon la revendication 1 ou 2, **caractérisé en ce qu'**au moins une des couches de recouvrement (21, 22) est fabriquée à partir d'une matière textile.

4. Article de compression selon l'une des revendications précédentes, **caractérisé en ce que** l'élément intermédiaire (23) est solidaire d'au moins une des couches de recouvrement (21, 22) ou est intégré dans au moins une des couches de recouvrement (21, 22).

5. Article de compression selon l'une des revendications précédentes, **caractérisé en ce que** l'élément intermédiaire (23) est fabriqué, au moins partiellement, à partir de monofilaments.

6. Article de compression selon la revendication 5, **caractérisé en ce que**, à l'état détendu de l'article de compression, les monofilaments sont disposés selon un angle compris entre 30° et 90° par rapport aux couches de recouvrement (21, 22).

7. Article de compression selon la revendication 5 ou 6, **caractérisé en ce que** tous les monofilaments sont fabriqués à partir de la même matière ou de matières différentes, de préférence en polyester, polyamide ou polyoléfine.

8. Article de compression selon l'une des revendications précédentes, **caractérisé en ce que** les fils de poils (23) sont au moins partiellement des fils élastiques.

9. Article de compression selon l'une des revendications précédentes, **caractérisé en ce que** l'espacement entre les deux couches de recouvrement (21, 22) varie sur la surface de l'article de compression (10, 30).

10. Article de compression selon la revendication 9, **caractérisé en ce que** le tricot d'espacement (20) présente une structure en forme de gaufre ou de losange en raison de l'espacement variable entre les deux couches de recouvrement (21, 22).

11. Article de compression selon l'une des revendications précédentes, **caractérisé en ce que** des fils de trame et/ou de chaîne sont insérés entre les couches de recouvrement (21, 22) ou sont attachés à au moins une couche de recouvrement.

12. Article de compression selon la revendication 11, **caractérisé en ce que** les fils de trame (21, 22) sont au moins partiellement des fils élastiques.

13. Article de compression selon l'une des revendications précédentes, **caractérisé en ce que** le tricot d'espacement (20) s'étend sur toute la circonférence de l'article de compression (10, 30).

14. Article de compression selon l'une des revendications précédentes, **caractérisé en ce que** la direction de la rangée de mailles des couches de recouvrement (21, 22) correspond à la direction circonférentielle de l'article de compression.

15. Article de compression selon l'une des revendications précédentes, **caractérisé en ce qu'**une pression de compression ou plage de pression de compression et une courbe de pression de compression prédéterminées peuvent être obtenues par un rapport prédéterminé entre les dimensions des parties du corps et les dimensions de l'article de compression.
